# FASCICULE DE BREVET EUROPEEN

(11) **EP 0 557 389 B1**
(45) Date de publication et mention de la délivrance du brevet: **21.09.1994**
(21) Numéro de dépôt: 91920476.8
(22) Date de dépôt: 08.11.1991
(51) Int. Cl.: A61K 31/545, A61K 9/20

(54) **PROCEDE DE PREPARATION PAR COMPRESSION DIRECTE DE COMPRIMES DE DERIVES DE L'ACIDE CEPHALOSPORANIQUE**
Verfahren zur Herstellung von direktkomprimierten, Cephalosporansäure Derivate enthaltendenTabletten
DIRECT COMPRESSION METHOD FOR PREPARING PILLS HAVING CEPHALOSPORANIC ACID DERIVATIVES

(30) Priorité: 15.11.1990 FR 9014210
(43) Date de publication de la demande: 01.09.1993
(73) Titulaire: RHONE-POULENC RORER S.A., 92165 Antony Cédex (FR)
(72) Inventeur: LALY, Jean-Louis, F-37100 Tours (FR); LOMBARDI, Roberto, F-78100 Saint-Germain-en-Laye (FR)
(74) Mandataire: Le Pennec, Magali
(86) Numéro de dépôt international: FR9100872
(87) Numéro de publication internationale: WO9208463

(56) Documents cités:
- STN International Information Services, data base: Chemical Abstracts, volume 112, No. 4, abstract No. 25681s, Columbus, 0hio US
- STN International Information Services, data base: Chemical Abstracts, volume 109, No. 12, abstract No. 98626a, Columbus, 0hio, US
- WPIL, accession No. 89-327676 (45), Derwent Publications Ltd, London, GB

## Description

La présente invention concerne une nouvelle forme pharmaceutique à base de céphalosporine. Elle concerne plus particulièrement une nouvelle forme pharmaceutique comprimée.

L'invention concerne tout particulièrement une nouvelle forme pharmaceutique comprimée à base d'un dérivé d'hémisynthèse de l'acide acylamino-7 céphalosporanique administrable par voie orale. Elle concerne de préférence les dérivés de l'acide acylamino-7 vinyl-3 céphalosporanique. Elle concerne surtout la mise en forme de l'acide (6R, 7R)-7-[(Z)-2-(amino-2 thiazolyl-4) carbomethoxy iminoacetamido-2] oxo-8 vinyl-3 thia-5 aza-1 bicyclo [4.2.0] octene-2 carboxylique-2. Cette céphalosporine est plus connue sous la dénomination commune internationale de Céfixime.

Les composés de la classe des céphalosporines présentent une activité antibiotique importante, leur utilisation est d'ailleurs réservée à des cas relativement graves lorsqu'une autre thérapie antibiotique s'est revélée non satisfaisante. Ces composés de part leur activité très importante ont amené certains pays à créer une législation très sévère pour leur production. Ainsi ce genre de composés ne peut être fabriqué et mis en forme que dans des salles spécifiques ceci afin d'éviter toute contamination croisée avec tout autre médicament.

Une autre difficulté de la mise en forme de ces principes actifs est due à leur coût. En effet ces produits sont obtenus par un procédé long demandant un grand nombre d'étapes de synthèse et se présentent donc lors de leur achat à un prix matière extrêmement élevé.

Il est donc indispensable, pour les deux raisons préalablement citées, de posséder une technique de mise en oeuvre évitant au maximum les pertes de produit.

En ce qui concerne les techniques de mise en forme pharmaceutique comprimée deux voies de préparation sont connues:
- une mise en forme par voie humide
- une mise en forme par compression directe.

Dans le premier cas c'est à dire pour la mise en forme par voie humide de principes actifs, il fallait prévoir la succession des étapes suivantes:
- pesée des principes actifs et des excipients
- émottage et tamisage des divers constituants de la formule finale
- mélange primaire du principe actif et des excipients avec vérification de l'homogénéité du mélange obtenu
- mouillage du mélange
- granulation
- séchage
- régularisation des grains
- dilution
- mélange final
- compression.

La succession de ces étapes demande la présence de huit types d'appareillages différents. L'ensemble de l'opération de mise en forme exige pour chacun de ces appareillages à cause de la toxicité du principe actif un nettoyage irréprochable. Le changement d'appareil augmente d'une part le coût de production d'une manière non négligeable, en effet les pertes ne peuvent être réduites à une valeur inférieure à 2 %, et empêche d'autre part tout automatisation du procédé. La protection de l'environnement et l'hygiène industrielle sont d'autant plus difficiles à contrôler que le nombre d'étapes de synthèse et d'appareillages est élevé. Le procédé par voie humide exige en plus une étape de séchage avec un risque de détérioration du produit loin d'être négligeable. L'ensemble de ces contraintes techniques auxquelles s'ajoutent les contraintes législatives en ce qui concerne l'environnement ont amené les industriels à abandonner la mise en forme par voie humide.

La seconde voie possible de préparation d'une forme pharmaceutique comprimée d'un principe actif consiste à réaliser une compression directe d'un mélange de poudres, mélange dans le cas de la présente invention constitué de la céphalosporine et des excipients.

Cette technique semblait aussi à proscrire dans le cadre de la présente invention vu la proportion élevée du principe actif par rapport aux excipients. Le procédé de compression directe s'applique généralement à des comprimés dont la dose de principe actif est inférieure à 100 mg et dont la proportion du principe actif dans le comprimé ne dépasse pas 25 %. Au delà de ce pourcentage, il est impossible de "noyer" le principe actif dans la masse des excipients et ainsi de s'affranchir des contraintes physiques et chimiques du principe actif.

De façon tout à fait étonnante il est apparu que dans le cadre de la présente invention, l'ensemble de ces problèmes ont été résolus en réalisant une compression directe d'un mélange contenant une céphalosporine et un ou plusieurs excipients dans une proportion pondérale du principe actif dans le comprimé variant entre 20 et 90 % et de préférence entre 30 et 70 %.

La céphalosporine utilisée dans la présente invention est de préférence un dérivé de l'acide acylamino-7 vinyl-3 céphalosporanique et tout particulièrement ledit dérivé est celui qui est commercialisé sous la dénomination de Cefixime.

Les excipients mis en oeuvre sont choisis parmi notamment :
- les carbonates de calcium
- les phosphates de calcium
- les sulfates de calcium
- les celluloses microcristallines
- les poudres de cellulose
- les fructoses
- les différentes formes de lactose
- les mannitols
- les sorbitols
- les amidons
- les amidons prégélifiés
- les sucres.

On peut également ajouter des agents lubrifiants comme le stéarate de magnésium, l'acide stéarique, le talc ou un polyéthylène glycol.

La granulométrie du principe actif qui est préférée pour la mise en oeuvre de la compression par voie sèche de la présente invention est la suivante:
- moins de 5 % de particules supérieures à 250 »m
- entre 55 et 95 % de particules comprises entre 250 et 90 »m
- entre 5 et 40 % de particules inférieures à 90 »m.

La densité du principe actif est de préférence comprise entre 0,6 et 0,9.

Le procédé de préparation consiste à peser les différents constituants, à les émotter, à réaliser un premier mélange avec l'ensemble des constituants mais sans l'agent lubrifiant puis on ajoute l'agent lubrifiant, on réalise le mélange final et on procède à la compression. Ce procédé utilise seulement quatre appareils ce qui apporte l'avantage de réduire les pertes en principe actif en dessous de 1 %.

Les comprimés obtenus par le procédé préalablement décrit, qui font aussi partie de l'invention présentent une friabilité et des critères de clivage meilleurs que les mêmes comprimés fabriqués par une compression par voie humide. Ils présentent une homogénéité de répartition du principe actif excellente.

L'invention sera plus complètement décrite à l'aide des exemples suivants qui ne doivent pas être considérés comme limitatifs de l'invention.

### Exemple 1

On met en oeuvre les matières suivantes :

| | |
|---|---|
| Céfixime trihydrate | 184,60 kg |
| Amidon prégélifié (Starch 1500) | 48,98 kg |
| Phosphate dicalcique dihydrate | 122,44 kg |
| Stéarate de magnésium | 2,03 kg |
| Cellulose microcristalline (Avicel PH 102) | 41,95 kg |

Le Cefixime trihydrate présente la granulométrie suivante:

| Dimension du tamis en » | pourcentage |
|---|---|
| 1400 | 0 |
| 1000 | 0,14 |
| 710 | 0,23 |
| 500 | 0,33 |
| 355 | 0,42 |
| 250 | 1,6 |
| 180 | 4,13 |
| 125 | 37,11 |
| 90 | 39,16 |
| Fond | 16,85 |

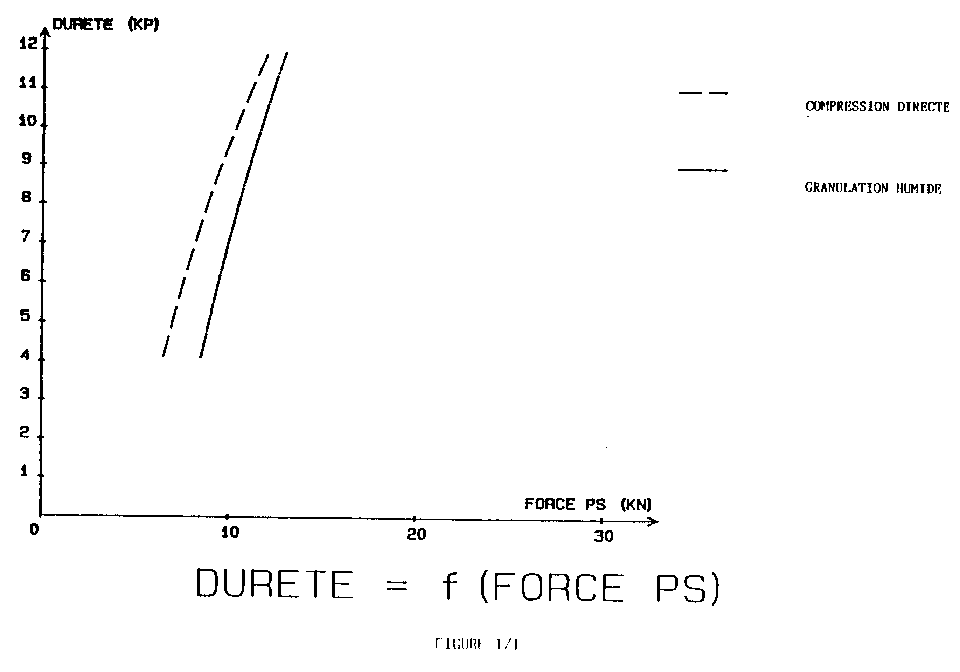

On passe les différentes matières, principe actif et excipient sur un tamis de grille 1mm pour réaliser un émottage.
Les matières sont pesées et chargées à l'exception du stéarate de magnésium dans un mélangeur de 1 000 litres. On effectue le mélange pendant 10 minutes à une vitesse de 10 tours par minute.
On ajoute le stéarate de magnésium préalablement émotté sur un tamis de 0,680 mm. On mélange 5 minutes à nouveau.
Pour comprimer on utilise une presse FETTE P 2000 ayant un nombre de station de 43. On produit 200 000 comprimés par heure. Le poids de comprimés fabriqués s'élève à 397 kg ce qui représente un rendement de production de 99,2 %.
Les comprimés ont un poids moyen de 491,5 mg avec une dispersion conforme à la Pharmacopée française. Le dosage du Cefixime effectué sur 15 comprimés varie de 192,7mg à 200,8 mg.
La dureté des comprimés a été mesurée de façon classique sur un appareil du type Schleuniger 4M sur 20 comprimés la mesure donne 100 à 140 newtons.
La comprimabilité du mélange a aussi été mesurée en fonction de la force appliquée sur le poinçon supérieur (exprimée en kilonewton) sur une chaîne d'extensométrie par un ensemble de capteurs qui mesurent la déformation des poinçons inférieurs et supérieurs et le déplacement du poinçon supérieur dans la matrice de la machine de compression.
Il a été établi des courbes comparatives qui expriment la comprimabilité du mélange. Ces courbes résultent de la mesure de la dureté des comprimés sur un appareil Schleuniger en fonction de la force appliquée en newtons sur le poinçon supérieur. Cette force est mesurée par une jauge de contrainte. Les courbes comparatives d'extensométrie ont été établies sur les comprimés obtenus par compression humide et par compression directe (voir Figure 1). La courbe obtenue montre que le simple mélange offre une meilleure réponse à la compression. Les comprimés obtenus par compression directe présentent une dureté nettement supérieure à ceux obtenus par granulométrie humide.

La friabilité de 20 comprimés mesurée sur un appareil ERWEKA modèle TAB après 10 minutes à 20 tours/minute est de 0,1 %.

Par ailleurs, l'historique industriel montre une constance des caractéristiques galéniques (dureté, friabilité, délitement, dissolution) des comprimés issus de différents lots de compression directe. Ce n'est pas le cas pour la granulation humide.

### EXEMPLE 2

On met en oeuvre les matières suivantes :

| | |
|---|---|
| Céfixime trihydrate | 46,666 kg |
| Amidon prégélifié (Starch 1500) | 7,000 kg |
| Hydrogénophosphate de calcium | 3,290 kg |
| Cellulose microcristalline | 12,624 kg |
| Stéarate de magnésium | 0,420 kg |

Le Cefixime trihydrate présente la granulométrie suivante:

| Dimension du tamis en » | pourcentage |
|---|---|
| 1400 | 0 |
| 1000 | 0,04 |
| 710 | 0,03 |
| 500 | 0,70 |
| 355 | 1,06 |
| 250 | 1,47 |
| 180 | 4,52 |
| 125 | 20,19 |
| 90 | 56,16 |
| Fond | 14,83 |
| Densité après tassement = 0,67 | |

On passe les différentes matières, principe actif et excipient sur un tamis de grille 0,8 mm d'ouverture de maille pour réaliser un émottage.
Les matières sont pesées et chargées à l'exception du stéarate de magnésium dans un mélangeur de 150 litres. On effectue le mélange pendant 10 minutes à une vitesse de 10 tours par minute. On ajoute le stéarate de magnésium préalablement émotté sur un tamis de 0,650 mm. On mélange 5 minutes à nouveau.
Pour comprimer on utilise une presse COURTOY R 100 ayant un nombre de station de 24. On produit 110 000 comprimés par heure.

Les comprimés ont un poids moyen de 596,3 mg avec une dispersion conforme à la Pharmacopée française. Le dosage du Cefixime effectué sur 20 comprimés varie de 394,4 mg à 407,12 mg. La dureté des comprimés mesurée avec l'appareil Schleuniger sur 20 comprimés varie de 100 à 140 N.

### EXEMPLE COMPARATIF 1

On met en oeuvre les matières suivantes:

| | |
|---|---|
| Céfixime trihydrate | 106,620 kg |
| Amidon prégélifié (Starch 1500) | 6,750 kg |
| Phosphate dicalcique dihydrate | 2,700 kg |
| Cellulose microcristalline (Avicel PH 102) | 7,868 kg |

On pèse les matières premières que l'on tamise et charge ensuite dans un mélangeur-granulateur de 400 litres. On mélange à sec pendant 5 minutes. On ajoute alors 24 kg d'eau puis progressivement 8,32 kg d'eau jusqu'à l'obtention d'une masse granulée qui contient 27 % d'eau. La granulation dure 7 minutes.
On sèche ensuite les granulés dans un sécheur à lit d'air fluidisé pendant 1 heure 15 minutes avec une température d'entrée d'air de 45°C et une température de sortie de 18°C - 25°C. Le granulé obtenu est ensuite passé sur un tamis de dimension de maille 1 mm. On obtient 119,938 kg de granulés.

On pèse ensuite:

| | |
|---|---|
| Cellulose microcristalline | 14,394 kg |
| Amidon prégélifié | 19,948 kg |
| Phosphate dicalcique dihydraté | 63,838 kg |
| Stéarate de magnésium | 0,886 kg |

On mélange les granulés obtenus précédemment avec la cellulose, l'amidon et le phosphate puis on ajoute le stéarate et on obtient 217,550 kg de produit final. Ce mélange est comprimée dans une presse FETTE P 2000. On obtient une masse de comprimés de 215,38 kg ce qui représente 98 % de rendement par rapport à la masse utilisée.
Les comprimés présentent un poids unitaire de 490 mg et une courbe d'extensométrie telle que reportée sur le diagramme en Figure 1. La friabilité des comprimés mesurée par la méthode TAB à 10 minutes est de 0,3 %. La dureté des comprimés varie de 85 à 95 N.

## Revendications

1. Procédé de préparation de comprimés de dérivés de l'acide acylamino-7 céphalosporanique caractérisé en ce qu'on réalise une compression directe d'un mélange contenant ledit acide en quantité pondérale de 20 à 90 % par rapport à la masse du comprimé.

2. Procédé de préparation selon la revendication 1 caractérisé en ce que le mélange utilisé pour la compression directe contient en poids 30 à 70 % dudit acide par rapport au poids du comprimé final.

3. Procédé de préparation selon la revendication 1 caractérisé en ce que l'acide acylamino-7 céphalosporanique présente une granulométrie telle que:
- moins de 5 % de particules ont un diamètre supérieur à 250 »
- entre 55 et 95 % des particules ont un diamètre compris entre 250 et 90 »
- entre 5 et 40 % des particules ont un diamètre inférieur à 90 ».

4. Procédé selon la revendication 1 caractérisé en ce que l'acide acylamino-7 céphalosporanique a une densité comprise entre 0,6 et 0,9.

5. Procédé selon la revendication 1 caractérisé en ce que le dérivé de l'acide acylamino-7 céphalosporanique est un dérivé de l'acide acylamino-7 vinyl-3 céphalosporanique.

6. Procédé selon la revendication 1 caractérisé en ce que les excipients sont choisis parmi:
- les carbonates de calcium
- les phosphates de calcium
- les sulfates de calcium
- les celluloses microcristallines
- les poudres de cellulose
- les fructoses
- les différentes formes de lactose
- les mannitols
- les sorbitols
- les amidons
- les amidons prégélifiés
- les sucres.

7. Procédé selon la revendication 6 caractérisé en ce que l'on ajoute un agent lubrifiant choisi parmi le stéarate de magnésium, l'acide stéarique, le talc et un polyéthylène glycol.

8. Comprimés obtenus selon l'une quelconque des revendications précédentes.

## Claims

1. Process for the preparation of tablets of derivatives of 7-acylaminocephalosporanic acid, characterized in that a direct compression is carried out of a mixture containing the said acid in a quantity of 20 to 90% by weight relative to the mass of the tablet.

2. Preparation process according to Claim 1, characterized in that the mixture used for the direct compression contains 30 to 70% by weight of the said acid relative to the weight of the final tablet.

3. Preparation process according to Claim 1, characterized in that the 7-acylaminocephalosporanic acid has a particle size such that:
- less than 5% of the particles have a diameter greater than 250 »m.
- between 55 and 95% of the particles have a diameter between 250 and 90 »m.
- between 5 and 40% of the particles have a diameter of less than 90 »m.

4. Process according to Claim 1, characterized in that the 7-acylaminocephalosporanic acid has a density of between 0.6 and 0.9.

5. Process according to Claim 1, characterized in that the 7-acylaminocephalosporanic acid derivative is a 7-acylamino-3-vinylcephalosporanic acid derivative.

6. Process according to Claim 1, characterized in that the excipients are chosen from:
- calcium carbonates
- calcium phosphates
- calcium sulphates
- microcrystalline celluloses
- cellulose powders
- fructoses
- the various forms of lactose
- mannitols
- sorbitols
- starches
- pregelatinized starches
- sugars.

7. Process according to Claim 6, characterized in that a lubricating agent chosen from magnesium stearate, stearic acid, talc and a polyethylene glycol is added.

8. Tablets obtained according to any one of the preceding claims.

## Patentansprüche

1. Verfahren zur Herstellung von Tabletten von 7-Acylaminocephalosporansäurederivaten, dadurch **gekennzeichnet,** daß man eine direkte Verpressung eines Gemisches, enthaltend diese Säure in einer Menge von 20 bis 90 Gew.-%, bezogen auf die Tablettenmasse, durchführt.

2. Verfahren zur Herstellung nach Anspruch 1, dadurch **gekennzeichnet,** daß das zur direkten Verpressung verwendete Gemisch 30 bis 70 Gew.-% der Säure, bezogen auf das Gewicht der Endtablette, enthält.

3. Verfahren zur Herstellung nach Anspruch 1, dadurch **gekennzeichnet,** daß die 7-Acylaminocephalosporansäure eine Korngrößenverteilung wie folgt besitzt:
- weniger als 5% der Teilchen besitzen einen Durchmesser größer als 250 »m,
- zwischen 55 und 95% der Teilchen besitzen einen Durchmesser zwischen 250 und 90 »m,
- zwischen 5 und 40% der Teilchen besitzen einen Durchmesser kleiner als 90 »m.

4. Verfahren nach Anspruch 1, dadurch **gekennzeichnet,** daß die 7-Acylaminocephalosporansäure eine Dichte zwischen 0,6 und 0,9 besitzt.

5. Verfahren nach Anspruch 1, dadurch **gekennzeichnet,** daß das 7-Acylaminocephalosporansäurederivat ein Derivat der 7-Acylamino-3-vinylcephalosporansäure ist.

6. Verfahren nach Anspruch 1, dadurch **gekennzeichnet,** daß die Excipientien ausgewählt sind aus:
- Calciumcarbonaten,
- Calciumphosphaten,
- Calciumsulfaten,
- mikrokristallinen Cellulosen,
- Cellulosepulvern,
- Fructosen,
- verschiedenen Lactoseformen,
- Manniten,
- Sorbiten,
- Stärken,
- vorgelierten Stärken,
- Zuckern.

7. Verfahren nach Anspruch 6, dadurch **gekennzeichnet,** daß man ein Gleitmittel, ausgewählt aus Magnesiumstearat, Stearinsäure, Talk, und ein Polyethylenglycol zusetzt.

8. Tabletten, erhalten nach einem der vorausgehenden Ansprüche.
